# EUROPEAN PATENT SPECIFICATION

(11) **EP 1 568 783 B1**
(45) Date of publication and mention of the grant of the patent: **11.07.2007**
(21) Application number: 04018374.1
(22) Date of filing: 03.08.2004
(51) Int. Cl.: C12Q 1/68

(54) **A nucleic acid based steganography system and application thereof**
Ein Nukleinsäure-basierendes steganografisches System und dessen Anwendung
Un système steganographique basés sur les acides nucléiques et application du même

(30) Priority: 06.08.2003 CN 03127517
(43) Date of publication of application: 31.08.2005
(73) Proprietor: Rixflex Holdings Limited, Road Town, Tortola (VG)
(72) Inventor: Liang, Benjamin, Chung-Ho City, Taipei Country (TW)
(74) Representative: Viering, Jentschura & Partner

(56) References cited:
- WO-A-00/68431
- WO-A-01/36676
- US-B1- 6 280 935

## Description

### BACKGROUND OF THE INVENTION

### 1.Field of the Invention

The present invention relates to a nucleic acid based steganographic technique, which can encipher and decipher predefined messages. In particular, the method of the invention relates to the division of the nucleic acid encoded message into a plurality of fragments, and then applying an encipherment procedure to these fragments through multiple encryption steps, which can be decoded with the corresponding deciphering method.

### 2. The Prior Arts

Information can be concealed through some special encipher techniques or secret keys to hide or transfer, which can be decoded only by authorized but not other people. The correct information can be deciphered with a private way to unlock the message.

Nucleic acid is the key information molecule in living system, since it carries the encipherment information that uniquely identifies each organism. Nucleic acid molecule is made of four bases to form a long chain macromolecule, the arrangement of bases is like a secret message in nature. The sequence of nucleic acid molecule can be used as a unique mark after some specific designs and treatments. The application can be broadened if special encryption technique is carried out in nucleic acid sequence. The nucleic acid marks can be placed inside edible materials, printed in tablets (such as in the forms of capsule, caplet, or lozenge) or surface of the pill provided by the health foods or pharmaceutical industries. In addition, nucleic acid marks can be labeled or hidden in the food-wrap (such as inner lining of aluminum boxes, covers of wrap box or wrap bottles).

Application of nucleic acid marks or taggants is very broad. For example, these nucleic acid marks are usually added to inks along with some other materials such as dyes, glue or resins to produce an anti-counterfeit nucleic acid-embedded ink. They can also be applied in patch behind the dollar patch, fluorescent printing, and microprinting to meet the needs of different products, which concealed the predefined messages in the printed product.

Nucleic acid based steganographic techniques have these advantages: very small amounts of nucleic acid are needed, low cost, and can be incorporated into ink for use in printing and applied in many printing businesses. For example, it can be used in company's important or classified documents; bank passbook or such as certificate of stocks, checks, bond papers of paper currency in financial industry; membership cards and coupons of department stores or clubs; painting or sculptures or other handmade crafts in arts; and other applications such as lottery drawing papers, stamps, custom labels, textile products, fibers, fabrics, staining dyes and so on.

Methods of using nucleic acid to encode hidden messages can also be applied in a living organisms or any organism to protect biotech products such as important plants, vaccines, and animals those with high economic values.

A producer or company can distinguish an object from real to fake through nucleic acid steganography, to tell if the object is from self-production or stolen pickings. Therefore, this technique can be used in anti-counterfeit or appraisal for counterfeits as well as hiding or transmitting some predefined messages.

There are several techniques of using nucleic acid sequences to encode hidden messages. For example, US Pat. Nos. 6,312,911 discloses a technique for DNA-based steganography. At first, The US patent creates an encryption key. According to this key, a secret-message DNA strand containing a message encoded in DNA is constructed and the real DNA is synthesized. The secret-message DNA strand is then concealed within the enormous complexity of fragmented human genomic DNA. In addition, the US patent further discloses a deciphering method. The secret-message DNA strand is flanked by primer sequences (DNA "tags"), which is a decryption key, known only to the sender and the intended recipient. The secret message can be recovered with the encryption key after employing knowledge of the primer sequences to specifically PCR-amplify the secret message DNA strand. And DNA sequence is determined through analysis.

However, calculating counterfeiters or people who intends to steal secrets can guess the primers needed for DNA amplification through the mature PCR technology to break the code directly or indirectly. For example, the random primers can be used to remove the noise and subtracting PCR technology can be employed to increase the probability of finding the secret codes by people who skilled in molecular biology. In addition, the whole gene sequences of several living organisms have been determined since year 2002, and the analysis function of computer becomes more and more powerful. The hidden messages can be revealed once the nucleotide sequences are compared and aligned with sequences in the gene bank.

To face the abovementioned problems, a more sophisticated method is necessary to protect the enciphered messages from being stolen during transmission or cryptanalysis process.

### SUMMARY OF THE INVENTION

To solve the problem of abovementioned technique, the inventors use a multiple encryption technique to assure that the secret messages are covered with several layers of security, and are difficult to decipher the hidden messages for business spies or counterfeiters.

Therefore, the primary object of the present invention is to provide a nucleic acid based encryption method to encode hidden messages. First of all, the hidden message is constructed according to a predetermined cipher table and synthesized as a secret message nucleic acid strand. This nucleic acid strand is hidden after divided into a plurality of fragments, which makes the probability of code breaking very small.

Procedures provided in the enciphering method of the present invention including:
(a) a predetermined "cipher table" is employed to transform a secret message into a corresponding original nucleic acid sequence;
(b) this original nucleic acid molecule is divided into a plurality of nucleic acid fragments, and then the nucleotide sequence of each nucleic acid fragment is obtained;
(c) predetermined oligomers for sequence analysis are ligated to each of the 5'- or 3'-end of the nucleic acid fragments described in step (b) respectively to become the first ligated products. These oligomers for sequence analysis will be applied to complement a sequencing primer during sequence analysis of deciphering process;
(d) At least one pair of predetermined oligomers for sequence recognition are ligated to each of the 5'- and 3'-end of the first ligated products obtained from step (c) respectively to become second ligated products. These pairs of oligomers for sequence recognition will be used to complement PCR primers during PCR reaction of deciphering process; and
(e) The second linked products are located inside a media and also concealed in that media.

Another object of the present invention is to provide a deciphering method corresponding to the abovementioned enciphering method.

Procedures provided in the deciphering method of the present invention comprise the steps of:
(i) isolating nucleic acid molecules from a media in a target desired to decipher;
(ii) performing a PCR reaction with a pair of primers corresponding to a predetermined oligomer for sequence recognition, which used in enciphering process, to obtain amplified PCR products containing a fragmented nucleotide;
(iii)performing a sequence analysis with a sequencing primer corresponding to a predetermined oligomer for sequence analysis, which used in enciphering process, to determine the fragmented nucleotide sequences of the PCR products obtained from step (ii);
(iv)determining the order of each fragmented nucleotide sequences;
(v) figuring out the original nucleotide sequence according to the informations of step (iii) step (iv);
(vi)deciphering the predefined message corresponded to the original nucleotide sequence after cryptanalysis on the predetermined cipher table.

The DNA molecule can be taken as an example. DNA is composed of four nucleotide bases, which includes adenine(A), guanine(G), cytosine(C), or thymine(T). People who are skilled in the art can arrange the bases in a specific order to represent a special meaning, in order to encode a secret message into a DNA sequence.

In the present invention, a secret message is encoded into an original nucleic acid sequence according to a predetermined cipher table, which makes the secret message concealed in the original nucleic acid sequence. This cipher table may be created by the users, the known Moss code or the encryption key described in US Pat. Nos. 6,312,911.

There are two ways of encryption process used in the present invention. Each DNA fragment may be artificially synthesized and be encrypted respectively. Or, the original DNA sequence may be synthesized in full length at first, and is divided into a plurality of nucleic acid fragments. Encryption of each fragment is carried out thereafter.

In addition, there is no limitation to the oligomers for sequence analysis used in encryption process. Oligomers for sequence analysis may be the same or different for each nucleic acid fragment and the oligomers may be ligated to either the 5'-end or the 3'-end of the nucleic acid fragments.

To increase the deciphered difficulty, at least one of the meaningless pseudo-sequences may be ligated after the oligomer for sequence analysis is ligated to the fragmented nucleic acid. There will be more information needed to decipher the secret message when there are more pseudo-sequences. The complicated the process is, the difficulty on the code breaking increases. Therefore, the security level will be assured with the multiple encryption process, which will decrease the probability of being deciphered to large extent.

On the other hand, the second ligated products may be inserted into vectors. These vectors for each second ligated products may be the same or different to each other. In addition, different second ligated products may be inserted into different sites of one vector if this vector is large enough. These message-containing vectors may be put into reserved media directly or after mixed with other genomic DNA to reduce the probability of being decoded.

Materials of the media described in the present invention are not specified, which include paper, glass, plastic, nitrocellulose layer, polycarbonic ester, nylon layer or textiles and so on. And the abovementioned second ligated products may be concealed into the same or different media.

In the process of decipherment, the decryption keys may be designed to be in multiple, different styles to increase the difficulty for code breaking according to the need of users.

Glossary of terms used in this invention is listed below to make explanation more clearly.

"Original nucleic acid sequence" indicates nucleic acid sequence encoding a message according to the meaning specified in a predetermined "cipher table".

"Fragmented DNA sequence" indicates a partial sequence obtained from original nucleic acid sequence after dividing.

"Oligomer for sequence analysis" indicates an oligomer, which can be used in analyzing sequence of the fragmented nucleic acid during decipherment process.

"First ligated product" indicates a ligated product of nucleic acid fragments, after being ligated with the oligomers for sequence analysis at each of the 5'- or 3'-end respectively.

"Oligomer for sequence recognition" indicates an oligomer that can be used for recognition of the sites of the fragmented nucleic acid and in amplification of PCR reaction.

"Second ligated product" indicates a ligated product of first ligated product, after being ligated with the oligomer for sequence recognition at both of the 5'- and 3'-end. The first ligated product may contain at least one of the pseudo-sequences after being ligated with the oligomer for sequence recognition.

"Pseudo-sequence" indicates a sequence without any meaning, which is applied to interfere with the analysis of the fragmented nucleic acid sequence or original nucleic acid sequence. The complexicity of decipherment is therefore being increased.

"Oligomer for order arrangement" indicates an oligomer that can be used in arranging the fragmented nucleic acid sequences in the right order during decipherment.

"Decryption key" indicates a necessary information for breaking the codes of original nucleic acid sequence.

The present invention is further explained in the following embodiment illustration and examples.

### BRIEF DESCRIPTION OF THE DRAWINGS

**FIG. 1** shows the diagram of division of original nucleic acid sequence into fragmented nucleic acid sequences.

**FIG. 2** shows the diagram of addition of 5'-end oligomer for sequence analysis including ligation of oligomer for sequence analysis, pseudo-sequences, 5'-end and 3'-end oligomers for sequence recognition, and cloning into a chosen vector. Dashed line represents pseudo-sequences; wave line represents the vector sequence.

**FIG. 3** the diagram of addition of 3'-end oligomer for sequence analysis including ligation of oligomer for sequence analysis, pseudo-sequences, 5'-end and 3'-end oligomers for sequence recognition, and cloning into a chosen vector. Dashed line represents pseudo-sequences; wave line represents the vector sequence.

**FIG. 4** shows the diagram of PCR reaction and PCR products after addition of first decryption key (complimentary sequence of oligomer for sequence recognition, PCR primer) during decipherment process.

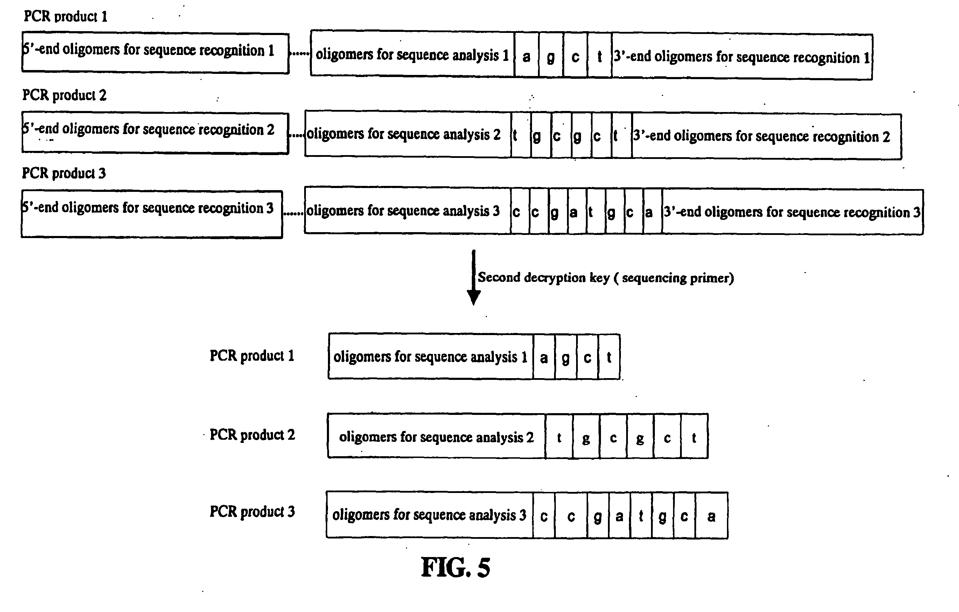

**FIG. 5** shows the diagram of sequence analysis on PCR products after addition of second decryption key (complimentary sequence of oligomer for sequence analysis, sequencing primer) during decipherment process.

**FIG. 6** shows the diagram of order arrangement using oligomers for order arrangement as the third decryption key.

**FIG. 7** shows the diagram of a decryption process using an oligomer for order arrangement as a third decryption key and a base frequency digital code as a fourth decryption key.

### DETAILED DESCRIPTION OF THE PREFERRED EMBODIMENT

The specific embodiments and preferred methods are described herein.

### Example 1: Encryption of a predefined message according to the methods described in the present invention

Now assume a producer wants to hide a taggant "MADE IN BWL" in one of his products to prevent the product being counterfeited. This producer can convert this message "MADE IN BWL" into an original DNA sequence according to an established cipher table. An example is taken for easy explanation: assume "MADE IN BWL" is encoded in a DNA sequence of "agcttgcgctccgatgca" (SEQ ID NO: 1) according to the cipher table.

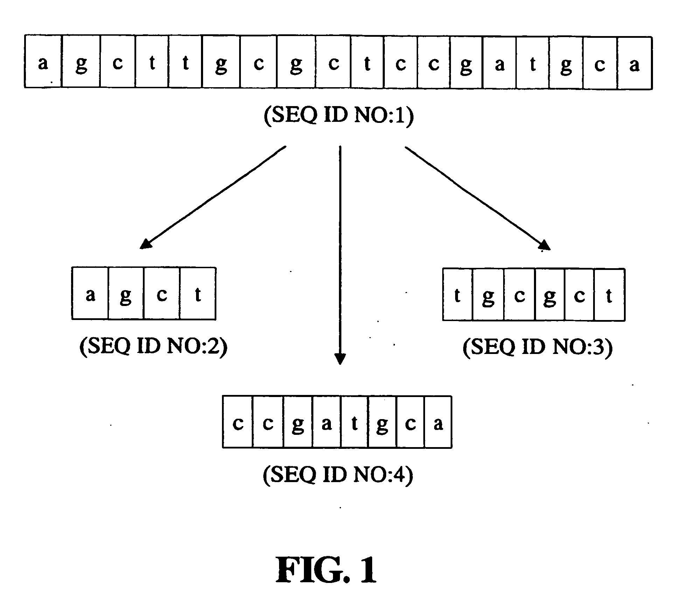

Next, as shown in **FIG. 1,** "agcttgcgctccgatgca" is divided into 3 pieces of fragmented DNA sequences, such as "agct" (SEQ ID NO: 2), "tgcgct" (SEQ ID NO:3), and "ccgatgca" (SEQ ID NO:4). There are two ways to obtain the fragmented DNA sequences. Each DNA fragments can be artificially synthesized from a DNA synthesizer respectively, or the original DNA sequence can be synthesized in full length and divided into the desired DNA fragments according to the known techniques.

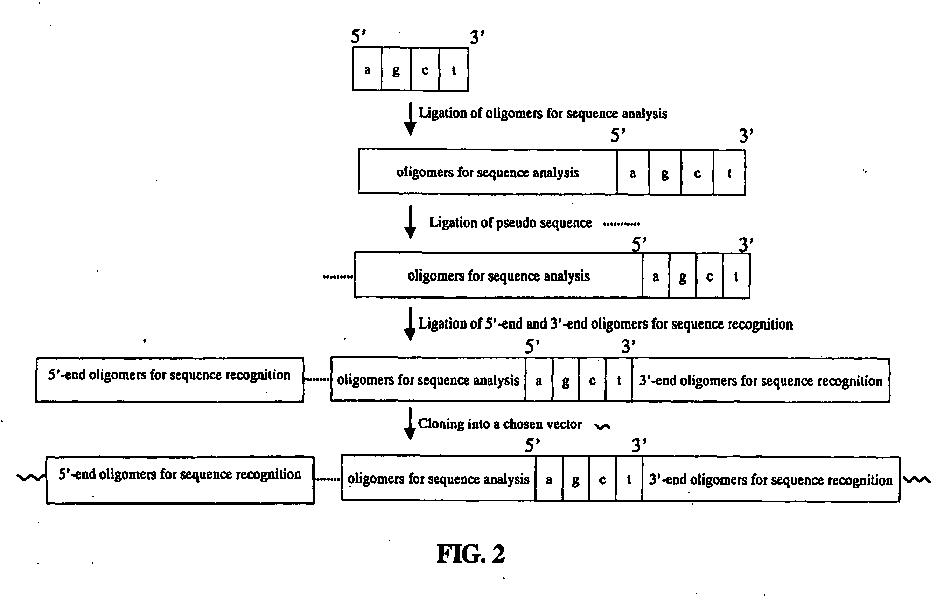

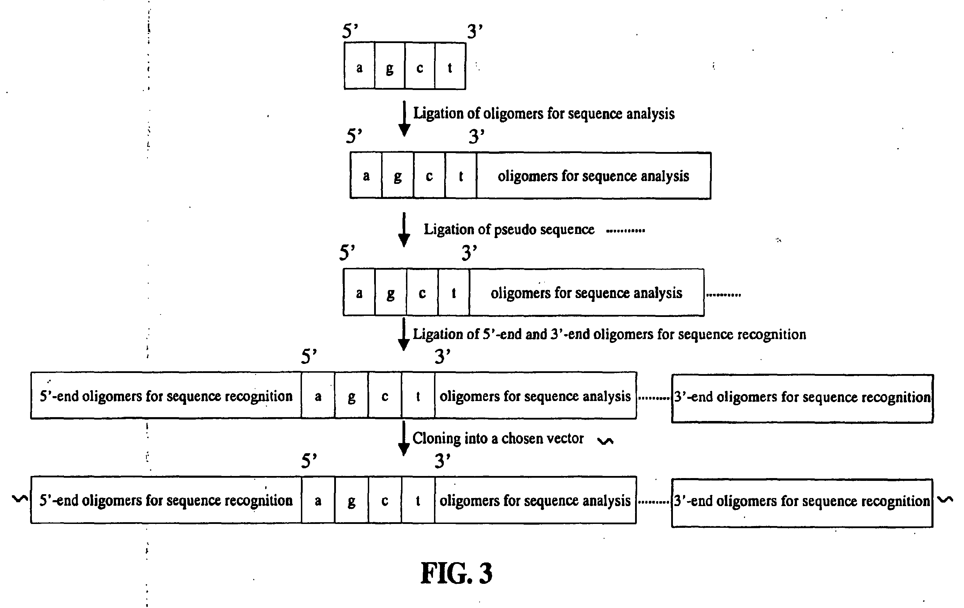

The fragmented DNA sequences are encrypted according to the following steps. As shown in **FIG. 2** or **FIG. 3**, an oligomer for sequence analysis "atcaatacttataatttggtt" (SEQ ID NO:5) is ligated to the 5'-end or 3'-end of fragmented DNA sequence (SEQ ID NO:2) to form a first ligated product. A pseudo-sequence may be ligated into the 5'-end (**FIG. 2**) or 3'-end (**FIG. 3**) of the first ligated product to increase the complexity of the original sequence and to prevent from being decoded. Next, a 5'-end oligomer for sequence recognition "gcgcgctaataactacacattta" (SEQ ID NO:6) as well as a 3'-end oligomer for sequence recognition "cccgggctcttatatatttcaattt" (SEQ ID NO : 7) are ligated to the first ligated product to obtain a second ligated product. In the following step, the second ligated product is cloned into a chosen vector. The rest of fragmented DNA sequences can be treated in the same way. And then all the second ligated products are pooled and mixed with a chosen media, and therefore further concealed the DNA-encoded message to a media.

In the abovementioned encryption process, each product can be amplified to the amount needed using PCR technology after ligation of 5'-end and 3'-end oligomer for sequence recognition. The products may be inserted into a DNA vector, as shown in **FIG. 2** and **FIG. 3**, through genetic engineering technology including application of restriction enzymes, or treatment of cohesive ends, and so on. These technologies are well known to person who is skilled in molecular biology and are routinely being performed.

It shall be realized that the original DNA sequence of 18 bases shown in above is an example used for brief explanation. In practice, the scope of the present invention is not limited by this example.

The fragmented DNA sequences, obtained after secret message DNA division, may be in length of 4 to 1000 bases, preferably in length of 20 to 500 bases, and most preferably in length of 50 to 150 bases.

The oligomers for sequence analysis, either be the same or different from each other, may be in length of 4 to 100 bases, preferably in length of 10 to 50 bases, and most preferably in length of 10 to 30 bases.

The pseudo-sequences may be in length of 4 to 100 bases, preferably in length of 10 to 50 bases, and most preferably in length of 10 to 30 bases.

The 5'-end or 3.'-end oligomers for sequence recognition, either be the same or different from each other, may be in length of 4 to 100 bases, preferably in length of 10 to 50 bases, and most preferably in length of 10 to 30 bases.

As described above, the encryption process of the present invention makes it very difficult to transform the encrypted data back to the original message when the informations of oligomers for sequence analysis, and 5'-end or 3'-end oligomers for sequence recognition are lack.

Such second ligated products or vectors carrying second ligated products may be mixed with non-interfering genomic DNA or randomly synthesized DNA to a large extent, and put the mixture into a media to increase the difficulty of code breaking techniques. Mixing them this way creates confusion, which makes the secret message even more difficult to be identified.

In comparison to the US patent of 6,312,911, which uses 3x10⁹ base pairs of human genome DNA to conceal the message and to decrease the probability of being decoded, the nucleic acid based steganography system used in the present invention is more practical and contains more confidentiality because the message can be divided into a plurality of short pieces and confused with other short pseudo-sequences.

### Example 2 Decryption according to the methods described in the present invention

The deciphering method used to break the abovementioned enciphering technique in example 1 is described below.

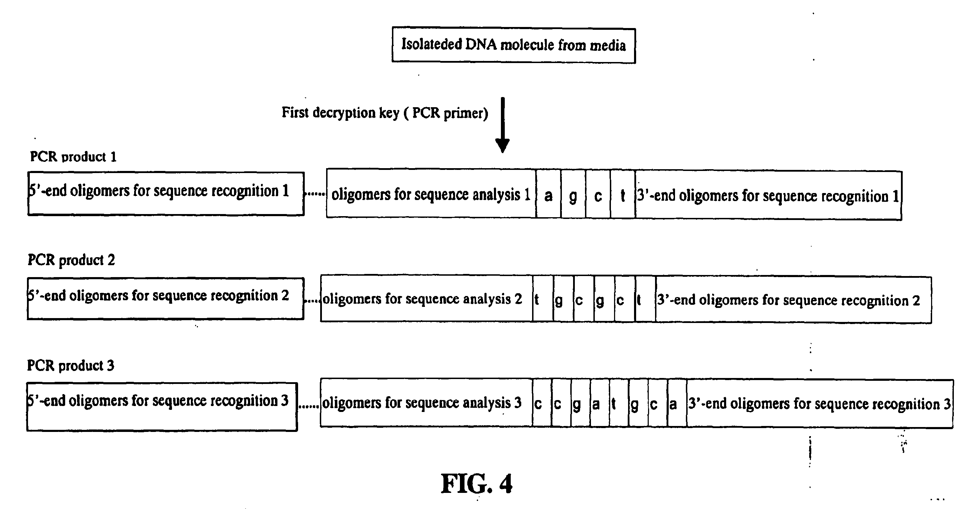

As shown in **FIG. 4,** the DNA molecules are isolated from media. The PCR primers, which are complementary to 5'-end or 3'-end oligomers for sequence recognition in Example 1, are employed in PCR reaction to fish out the secret message DNA from the DNA pools. These PCR primers are the first decryption keys, and the amplified products are PCR product 1, PCR product 2 and PCR product 3.

Sequence analysis of PCR product 1, PCR product 2 and PCR product 3 are carried out with sequencing primers, which are complimentary to oligomers for sequence analysis in Example 1. These sequencing primers are the second decryption keys, and the sequencing reaction is performed either with a traditional DNA sequencer or with Pyrosequence analysis.

What needs to be understood is that, the PCR products, including PCR product 1, PCR product 2 and PCR product 3, contain the sequences of 3'-end oligomers for sequence recognition, fragmented DNA sequences which are partial sequences of the original DNA sequences, oligomers for sequence analysis, pseudo-sequences and 5'-end oligomers for sequence recognition. The fragmented DNA sequences can be determined when all the informations of oligomers for sequence analysis, pseudo-sequences and 5'-end oligomers for sequence recognition are removed. In addition, other sequences will not be analyzed if the sequence analysis is stopped till the end of fragmented DNA sequence or oligomers of sequence recognition. And only the information of oligomers for sequence recognition is needed to be removed to obtain the sequence of fragmented DNA.

The fragmented DNA sequences should be put in the right order to solve the complete original DNA sequence after the sequences of fragmented DNA are known. The oligomers for order arrangement are created with predetermined rules derived from original DNA sequence and therefore are used as additional decryption keys to break the codes during deciphering process.

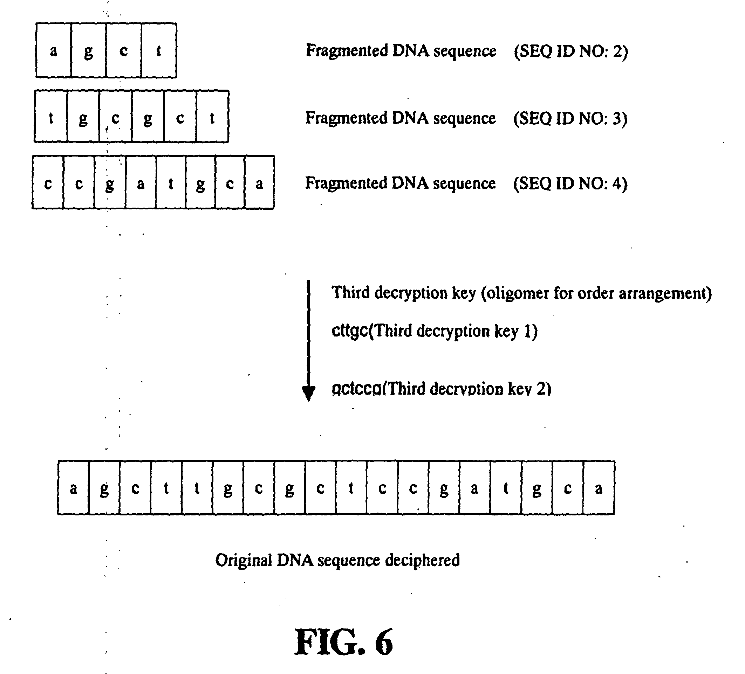

As shown in **FIG. 6**, the number of oligomers for order arrangement is one less than the number of DNA fragments. The oligomers for order arrangement, which are used as a third decryption keys, are applied to ensure the order of each DNA fragments. The encryption keys may be made as described in the following example: the 2 bases from the 3'-end of SEQ ID NO: 2 is combined with 3 bases from the 5'-end of SEQ ID NO: 3 to generate a third decryption key 1 "cttgc"(SEQ ID NO:8), and the 3 bases from the 3'-end of SEQ ID NO: 3 is combined with 3 bases from the 5'-end of SEQ ID NO: 4 to generate a third decryption key 2 "gctccg"(SEQ ID NO: 9). Therefore, people knows the information of the third decryption keys can arrange the fragmented DNA sequences in the right order during decipherment to obtain the secret encoded message.

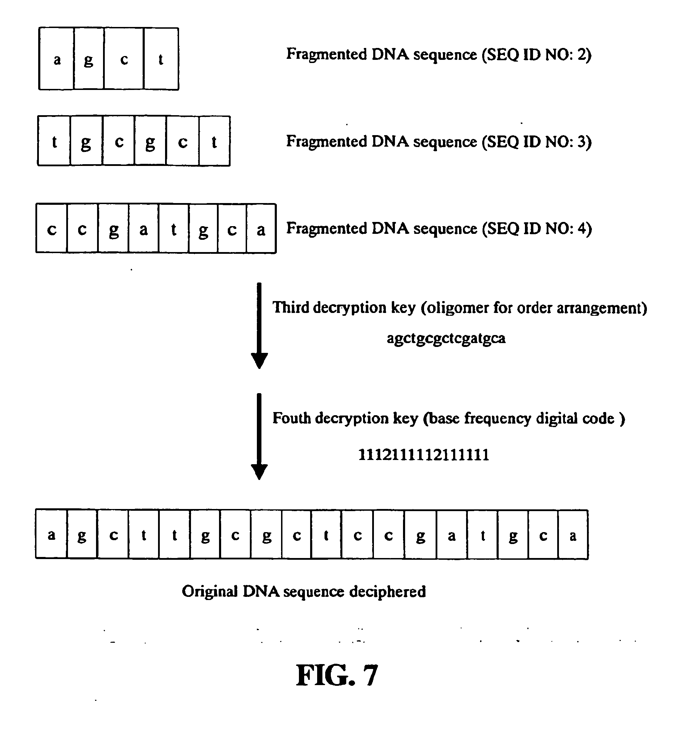

Another design of order arrangement is shown in **FIG. 7**. The repeated bases in the DNA strand in the third encryption key 3 "agctgcgctcgatgca" (SEQ ID NO: 10) are shown once only. The frequency of each base displayed in the original DNA sequence is shown correspondingly in digital code of base frequency as a fourth decryption key "1112111112111111". The digital code showed in **FIG. 7** represents that the frequency of the fourth and the tenth bases are in duplicate while other bases show once only. From this way, the order and the frequency of the bases can be postulated during decipherment to obtain the secret encoded message.

When the original DNA sequence is determined, the secret message corresponded to the DNA strand is deciphered after cryptanalysis on the predetermined cipher table.

As described in the above explanation and examples, the oligomers for sequence analysis and oligomers for sequence recognition in the present invention are designed beforehand. Therefore, there is no way to carry out the PCR reaction and sequence analysis when those oligomers are unknown. The concealed secret message can not be resolved without knowing the sequencing primers although intended people uses random primers to synthesize DNA randomly or from genomic DNA.

In addition, the code breaker need to know all the sequences of oligomers for sequence analysis and oligomers for sequence recognition to decipher the original DNA sequence because the full length DNA is divided into several fragments. And even the abovementioned oligomers are known, the original DNA sequence still can not be solved if the oligomers for order arrangement are not known. Therefore, the security level is assured with these multiple encryption process in comparison to the previous DNA steganographic technique.

On the other hand, the enciphering and deciphering methods provided by the present invention can be applied in the management of supplying chain, including different levels of management in merchandises production, logistic and quality control. For example, the headquarters can design a specific DNA-based message and divide it into different pieces. These DNA pieces can be put into products or semi-manufactures of different stages. The first decryption key can be distributed to a quality control unit, to analyze if particular PCR product existed, and to find the problematic process.

In addition, the first decryption keys initially can be used to carry out a PCR reaction to distinguish genuine products from counterfeit products immediately. For confirmation, the second decryption keys can be used to learn the fragmented DNA sequences. Finally, the third decryption keys, or even the fourth decryption keys can be used to solve the order of each DNA fragment to further confirm the product is real.

Those examples above should not, however, be considered to limit the scope of the invention, which is only defined by the scope of the appended claims.

## Claims

1. A nucleic acid based encryption method comprising the steps of:
(a) transforming a predefined message into a corresponding original nucleic acid sequence according to a predetermined cipher table;
(b) dividing the original nucleic acid sequence into a plurality of fragments, and obtaining the fragmented nucleic acid sequences of each fragment;
(c) ligating at least one predetermined oligomer for sequence analysis to each of the 5'- or 3'-end of the fragmented nucleotide sequences to become first ligated products, and the oligomers for sequence analysis are applied to complementing to sequencing primers during sequence analysis of deciphering process;
(d) ligating at least one pair of predetermined oligomers for sequence recognition to each of the 5'- and 3'-end of the first ligated products from step (c) to become second ligated products, and the pairs of oligomers for sequence recognition are used to complement to PCR primers during PCR reaction of deciphering process; and
(e) locating the second ligated products from step (d) inside a media and also concealed in the media.

2. The method according to claim 1, wherein the step (b) comprises synthesizing the fragmented nucleotide sequences.

3. The method according to claim 1, wherein the step (b) comprises synthesizing the full length of original nucleotide sequence, and dividing the original sequence into desired fragments.

4. The method according to claim 1, wherein the oligomers for sequence analysis, which are ligated to fragmented nucleotide sequence in step (c), are the same to each other.

5. The method according to claim 1, further comprises ligating at least one pseudo-sequence to at least one end of the first ligated products in step (c).

6. The method according to claim 1, further comprises cloning the second ligated products in step (d) into nucleotide vectors.

7. The method according to claim 6, wherein the nucleotide vectors to clone the second ligated products are the same to each other.

8. The method according to claim 6, wherein the nucleotide vectors to clone the second ligated products are different from each other.

9. The method according to claim 1, further comprises mixing the second ligated products in step (d) with genomic DNAs.

10. The method according to claim 1, wherein the media is selected from the group consisting of paper, glass, plastic, nitrocellulose layer, polycarbonic ester, nylon layer and textiles

11. A nucleic acid based decryption system comprising the steps of:
(i) isolating nucleic acid molecules from a media in a target desired to decipher;
(ii) performing a PCR reaction with a pair of primers corresponding to a predetermined oligomer for sequence recognition, which used in enciphering process, to obtain amplified PCR products containing a fragmented nucleotide;
(iii)performing a sequence analysis with a sequencing primer corresponding to a predetermined oligomer for sequence analysis, which used in enciphering process, to determine the fragmented nucleotide sequences of the PCR products obtained from step (ii);
(iv)determining the order of each fragmented nucleotide sequences;
(v) figuring out the original nucleotide sequence according to the informations of step (iii) step (iv);
(vi)deciphering the predefined message corresponded to the original nucleotide sequence after cryptanalysis on the predetermined cipher table.

12. The method according to claim 11, wherein the step (iv) comprises obtaining informations for determining the order of each fragmented nucleotide sequence comprising at least one of predetermined oligomers for order arrangement.

13. The method according to claim 12, wherein the oligomers for order arrangement are designed according to predetermined rules derived from the original nucleotide sequence.

14. The method according to claim 13, wherein the oligomers for order arrangement are produced according to the steps of:
(A) obtaining a plurality of bases in the 3'-end of a former fragmented nucleotide sequence between two adjacent fragmented nucleotide sequences;
(B) obtaining a plurality of bases in the 5'-end of a latter fragmented nucleotide sequence between two adjacent fragmented nucleotide sequences;
(C) combining the bases obtained from step (A) and step (B) in order to form an oligomer for order arrangement;
(D) repeating the steps from (A) to (C), till all the oligomers for order arrangement are constructed;
wherein, the number of oligomers for order arrangement is one less than the number of fragmented nucleotide sequences, and the direction of sequence arrangement is from 5'-end to 3'-end.

15. The method according to claim 12, further comprises obtaining at least one predetermined base frequency digital code indicating a base displayed frequency of the original nucleic acid sequence.

16. The method according to claim 15, wherein the oligomers for order arrangement are nucleotide sequences omitting repeated bases in the original nucleotide sequence.

17. The method according to claim 11, wherein the media is selected from the group consisting of paper, glass, plastic, nitrocellulose layer, polycarbonic ester, nylon layer and textiles.

## Patentansprüche

1. Auf Nukleinsäure basierendes Verschlüsselungsverfahren, das die Schritte umfasst:
(a) Umwandeln einer vordefinierten Nachricht in eine entsprechende Original-Nukleinsäuresequenz gemäß einer vorbestimmten Chiffriertabelle,
(b) Teilen der Original-Nukleinsäuresequenz in eine Vielzahl von Bruchstücken und erhalten der gestückelten Nukleinsäuresequenzen jedes Bruchstücks,
(c) Ligieren mindestens eines vorbestimmten Oligomers zur Sequenzanalyse an jedes der 5'- oder 3'-Enden der gestückelten Nukleotidsequenzen, um zu ersten ligierten Produkten zu werden, und die Oligomere zur Sequenzanalyse werden während einer Sequenzanalyse des Dechiffrierungsprozesses einem Komplementieren an Sequenzierungsprimer unterzogen,
(d) Ligieren mindestens eines Paars von vorbestimmten Oligomeren zur Sequenzerkennung an jedes der 5'- und 3'-Enden des ersten ligierten Produktes aus Schritt (c), um zu zweiten ligierten Produkten zu werden, und die Paare von Oligomeren zur Sequenzerkennung werden verwendet, um während der PCR-Reaktion des Dechiffrierungsprozesses an PCR-Primer zu komplementieren, und
(e) Einbringen des zweiten ligierten Produktes aus Schritt (d) in ein Medium, und auch Verbergen im Medium.

2. Verfahren nach Anspruch 1, wobei Schritt (b) das Synthetisieren der gestückelten Nukleotidsequenzen umfasst.

3. Verfahren nach Anspruch 1, wobei Schritt (b) das Synthetisieren der vollen Länge der Original-Nukleotidsequenz und Aufteilen der Originalsequenz in gewünschte Bruchstücke umfasst.

4. Verfahren nach Anspruch 1, wobei die Oligomere für eine Sequenzanalyse, die in Schritt (c) an die gestückelte Nukleotidsequenz ligiert werden, einander gleichen.

5. Verfahren nach Anspruch 1, das zusätzlich das Ligieren mindestens einer Pseudosequenz an mindestens ein Ende der ersten ligierten Produkte in Schritt (c) umfasst.

6. Verfahren nach Anspruch 1, das zusätzlich das Klonieren der zweiten ligierten Produkte in Schritt (d) in Nukleotidvektoren umfasst.

7. Verfahren nach Anspruch 6, wobei die Nukleotidvektoren, zum Klonieren der zweiten ligierten Produkte, einander gleichen.

8. Verfahren nach Anspruch 6, wobei die Nukleotidvektoren, zum Klonieren der zweiten ligierten Produkte, voneinander verschieden sind.

9. Verfahren nach Anspruch 1, das zusätzlich das Mischen der zweiten ligierten Produkte in Schritt (d) mit genomischen DNAs umfasst.

10. Verfahren nach Anspruch 1, wobei das Medium aus der Gruppe ausgewählt wird, die aus Papier, Glas, Plastik, einer Nitrozelluloseschicht, Polykarbonatester, einer Nylonschicht und Textilien besteht.

11. Auf Nukleinsäure basierendes Dechiffrierungsverfahren, das die Schritte umfasst:
(i) Isolieren von Nukleinsäuremolekülen aus einem Medium in einem Ziel, die zu dechiffrieren gewünscht ist,
(ii) Durchführen einer PCR-Reaktion mit einem Paar an Primern, die einem vorbestimmten Oligomer für eine Sequenzerkennung entsprechen, das in einem Verschlüsselungsverfahren verwendet wurde, um amplifizierte PCR-Produkte zu erhalten, die ein gestückeltes Nukleotid enthalten,
(iii) Durchführen einer Sequenzanalyse mit einem Sequenzierungsprimer, der einem vorbestimmten Oligomer für eine Sequenzanalyse entspricht, der in einem Verschlüsselungsverfahren verwendet wurde, um die gestückelten Nukleotidsequenzen des PCR-Produktes zu bestimmen, das aus Schritt (ii) erhalten worden ist,
(iv) Bestimmen der Reihenfolge jeder der gestückelten Nukleotidsequenzen,
(v) Herausfinden der Original-Nukleotidsequenz gemäß der Informationen aus Schritt (iii) Schritt (iv)
(vi) Dechiffrieren der vordefinierten Nachricht, die der Originalnukleotidsequenz entsprach, nach Verschlüsselungsanalyse auf der vorbestimmten Chiffriertabelle.

12. Verfahren nach Anspruch 11, wobei der Schritt (iv) das Erhalten von Informationen zum Bestimmen der Reihenfolge jeder gestückelten Nukleotidsequenz umfasst, was mindestens eines von vorbestimmten Oligomeren zur Anordnung der Reihenfolge umfasst.

13. Verfahren nach Anspruch 12, wobei die Oligomere zur Anordnung der Reihenfolge nach vorbestimmten Regeln gestaltet sind, die aus der Original-Nukleotidsequenz hergeleitet sind.

14. Verfahren nach Anspruch 13, wobei die Oligomere zur Anordnung der Reihenfolge gemäß den Schritten hergestellt sind:
(A) Erhalten einer Mehrzahl von Basen am 3'-Ende einer vorherigen gestückelten Nukleotidsequenz zwischen zwei aneinander angrenzenden gestückelten Nukleotidsequenzen,
(B) Erhalten einer Mehrzahl von Basen am 5'-Ende einer letzteren gestückelten Nukleotidsequenz zwischen zwei aneinander angrenzenden gestückelten Nukleotidsequenzen,
(C) Kombinieren der aus Schritt (A) und Schritt (B) erhaltenen Basen, um ein Oligomer zur Anordnung der Reihenfolge zu bilden,
(D) Wiederholen der Schritte von (A) bis (C), bis alle Oligomere zur Anordnung der Reihenfolge erstellt sind,
wobei die Zahl der Oligomere zur Anordnung der Reihenfolge um eins kleiner ist als die Zahl der gestückelten Nukleotidsequenzen und die Richtung der Sequenzanordnung vom 5'-Ende zum 3'-Ende ist.

15. Verfahren nach Anspruch 12, das zusätzlich das Erhalten mindestens eines vorbestimmten Basenhäufigkeitscodes umfasst, der die von einer Base gezeigte Häufigkeit in der Originalnukleotidsequenz anzeigt.

16. Verfahren nach Anspruch 15, wobei die Oligomere zur Anordnung der Reihenfolge Nukleotidsequenzen sind, wobei die sich wiederholenden Basen in der Originalnukleotidsequenz weggelassen werden.

17. Verfahren nach Anspruch 11, wobei das Medium aus der Gruppe ausgewählt wird, die aus Papier, Glas, Plastik, einer Nitrozelluloseschicht, Polykarbonatester, einer Nylonschicht und Textilien besteht.

## Revendications

1. Procédé de cryptage à base d'acide nucléique comprenant les étapes consistant à :
(a) transformer un message prédéfini en une séquence d'acide nucléique originelle correspondante conformément à un tableau de chiffrage prédéterminé ;
(b) diviser la séquence d'acide nucléique originelle en une pluralité de fragments, et obtenir les séquences d'acides nucléiques fragmentées de chaque fragment ;
(c) ligaturer au moins un oligomère prédéterminé pour une analyse de séquence à chacune des extrémités 5' ou 3' des séquences nucléotidiques fragmentées pour en faire des premiers produits ligaturés, et les oligomères pour l'analyse de séquence sont soumis à la complémentation avec des amorces de séquençage pendant l'analyse de séquences du processus de décryptage ;
(d) ligaturer au moins une paire d'oligomères prédéterminés pour la reconnaissance de séquence à chacune des extrémités 5' et 3' des premiers produits ligaturés de l'étape (c) pour en faire des seconds produits ligaturés, et les paires d'oligomères pour la reconnaissance de séquence sont utilisées pour complémenter les amorces de PCR pendant une réaction de PCR du processus de décryptage ; et
(e) localiser les seconds produits ligaturés de l'étape (d) à l'intérieur d'un milieu et également dissimulés dans le milieu.

2. Procédé selon la revendication 1, dans lequel l'étape (b) comprend la synthèse des séquences nucléotidiques fragmentées.

3. Procédé selon la revendication 1, dans lequel l'étape (b) comprend la synthèse de la longueur totale de la séquence nucléotidique originelle, et la division de la séquence originelle en fragments désirés.

4. Procédé selon la revendication 1, dans lequel les oligomères pour l'analyse de séquence, qui sont ligaturés à la séquence nucléotidique fragmentée dans l'étape (c), sont identiques les uns aux autres.

5. Procédé selon la revendication 1, comprenant en outre la ligature d'au moins une pseudo-séquence à au moins une extrémité des premiers produits ligaturés dans l'étape (c).

6. Procédé selon la revendication 1, comprenant en outre le clonage des seconds produits ligaturés dans l'étape (d) dans des vecteurs nucléotidiques.

7. Procédé selon la revendication 6, dans lequel les vecteurs nucléotidiques pour cloner les seconds produits ligaturés sont identiques les uns aux autres.

8. Procédé selon la revendication 6, dans lequel les vecteurs nucléotidiques pour cloner les seconds produits ligaturés sont différents les uns des autres.

9. Procédé selon la revendication 1, comprenant en outre le mélange des seconds produits ligaturés dans l'étape (d) avec des ADN génomiques.

10. Procédé selon la revendication 1, dans lequel le milieu est choisi dans le groupe consistant en papier, verre, plastique, couche de nitrocellulose, ester polycarbonique, couche de nylon et textiles.

11. Système de décryptage à base d'acide nucléique, comprenant les étapes consistant à :
(i) isoler des molécules d'acide nucléique à partir d'un milieu dans une cible que l'on souhaite décrypter ;
(ii) effectuer une réaction de PCR avec une paire d'amorces correspondant à un oligomère prédéterminé pour la reconnaissance de séquences, qui est utilisé dans un procédé de cryptage, pour obtenir des produits de PCR amplifiés contenant un nucléotide fragmenté ;
(iii) effectuer une analyse de séquence avec une amorce de séquençage correspondant à un oligomère prédéterminé pour l'analyse de séquence, qui est utilisé dans un procédé de cryptage, afin de déterminer les séquences nucléotidiques fragmentées des produits de PCR obtenus dans l'étape (ii) ;
(iv) déterminer l'ordre de chaque séquence nucléotidique fragmentée ;
(v) déterminer la séquence nucléotidique originelle d'après les informations de l'étape (iii) à l'étape (iv) ;
(vi) décrypter le message prédéfini correspondant à la séquence nucléotidique originelle après cryptanalyse sur le tableau de chiffrage prédéterminé.

12. Procédé selon la revendication 11, dans lequel l'étape (iv) comprend l'obtention d'informations pour déterminer l'ordre de chaque séquence nucléotidique fragmentée comprenant au moins un des oligomères prédéterminés pour un arrangement dans l'ordre.

13. Procédé selon la revendication 12, dans lequel les oligomères pour l'arrangement dans l'ordre sont conçus selon des règles prédéterminées dérivées de la séquence nucléotidique originelle.

14. Procédé selon la revendication 13, dans lequel les oligomères pour l'arrangement dans l'ordre sont produits selon les étapes consistant à :
(A) obtenir une pluralité de bases dans l'extrémité 3' d'une précédente séquence nucléotidique fragmentée entre deux séquences nucléotidiques fragmentées adjacentes ;
(B) obtenir une pluralité de bases dans l'extrémité 5' d'une séquence nucléotidique fragmentée suivante entre deux séquences nucléotidiques fragmentées adjacentes ;
(C) combiner les bases obtenues dans l'étape (A) et dans l'étape (B) afin de former un oligomère pour l'arrangement dans l'ordre ;
(D) répéter les étapes de (A) à (C), jusqu'à ce que tous les oligomères pour l'arrangement dans l'ordre soient construits ;
dans lequel le nombre d'oligomères pour l'arrangement dans l'ordre est inférieur d'une unité au nombre de séquences nucléotidiques fragmentées, et la direction de l'arrangement des séquences est de l'extrémité 5' vers l'extrémité 3'.

15. Procédé selon la revendication 12, qui comprend en outre l'obtention d'au moins un code numérique de fréquence de bases prédéterminé indiquant une fréquence de bases présentée de la séquence d'acide nucléique originelle.

16. Procédé selon la revendication 15, dans lequel les oligomères pour l'arrangement dans l'ordre sont des séquences nucléotidiques omettant des bases répétées dans la séquence nucléotidique originelle.

17. Procédé selon la revendication 11, dans lequel le milieu est choisi dans le groupe consistant en papier, verre, plastique, couche de nitrocellulose, ester polycarbonique, couche de nylon et textiles.


### SEQUENCE LISTING

<110> Biowell Technology Inc.
<120> A NOVEL NUCLEIC ACID BASED STEGANOGRAPHY SYSTEM AND APPLICATION THEREOF
<130> NP-17231-TWN
<150> TW092121490
   <151> 2003-08-06
<160> 10
<170> Patent In version 3.2
<210> 1
   <211> 18
   <212> DNA
   <213> Artificial
<220>
   <223> An example original DNA sequence corresponding to a message "MADE IN BWL"
<400> 1
   agcttgcgct ccgatgca 18
<210> 2
   <211> 4
   <212> DNA
   <213> Artificial
<220>
   <223> A fragmented DNA sequence from SEQ ID NO: 1
<400> 2
   agct 4
<210> 3
   <211> 6
   <212> DNA
   <213> Artificial
<220>
   <223> A fragmented DNA sequence from SEQ ID NO: 1
<400> 3
   tgcgct 6
<210> 4
   <211> 8
   <212> DNA
   <213> Artificial
<220>
   <223> A fragmented DNA sequence from SEQ ID NO: 1
<400> 4
   ccgatgca 8
<210> 5
   <211> 21
   <212> DNA
   <213> Artificial
<220>
   <223> An example oligomer for sequence analysis
<400> 5
   atcaatactt ataatttggt t 21
<210> 6
   <211> 23
   <212> DNA
   <213> Artificial
<220>
   <223> An example 5'-end oligomer for sequence recognition
<400> 6
   gcgcgctaat aactacacat tta 23
<210> 7
   <211> 25
   <212> DNA
   <213> Artificial
<220>
   <223> An example 3'-end oligomer for sequence recognition
<400> 7
   cccgggctct tatatatttc aattt 25
<210> 8
   <211> 5
   <212> DNA
   <213> Artificial
<220>
   <223> An example oligomer for order arrangement as a third decryption key (third decryption key number 1)
<400> 8
   cctgc 5
<210> 9
   <211> 6
   <212> DNA
   <213> Artificial
<220>
   <223> An example oligomer for order arrangement as a third decryption key (third decryption key number 2)
<400> 9
   gctccg 6
<210> 10
   <211> 16
   <212> DNA
   <213> Artificial
<220>
   <223> An example oligomer for order arrangement as a third decryption key
<400> 10
   agctgcgctc gatgca 16